# EUROPEAN PATENT APPLICATION

(11) **EP 0 694 610 A1**
(43) Date of publication of application: **31.01.1996**
(21) Application number: 95906521.0
(22) Date of filing: 26.01.1995
(51) Int. Cl.: C12N 1/16, A23K 1/18

(54) **COATED $i(PHAFFIA RHODOZYMA) YEAST AND GRANULE THEREOF**

(30) Priority: 26.01.1994 JP 23628/94
(71) Applicant: KANEGAFUCHI KAGAKU KOGYO KABUSHIKI KAISHA, Kita-ku Osaka-shi Osaka-fu 530 (JP)
(72) Inventor: UEDA, Yasuyoshi, Hyogo-ken 671-12 (JP); IMAMURA, Shiro, Kakogawa-shi Hyogo-ken 675 (JP)
(74) Representative: VOSSIUS & PARTNER
(86) International application number: JP9500088
(87) International publication number: WO9520647

(57) **Abstract**

*Phaffia rhodozyma* yeast dried in the presence of a sugar and containing astaxanthin in a stabilized state. It can prevent astaxanthin from being decomposed and provides a high granulation strength.

## Description

### FIELD OF THE INVENTION

The present invention relates to astaxanthin-producing *Phaffia rhodozyma* yeast which is not only useful for coloration of the meat or skin of salmon, trout, red sea bream, goldfish and prawn as well as feather of red canary, but also effective for the reinforcement of nutritive values.

### BACKGROUND OF THE INVENTION

In recent years, cultivation of fishes such as salmon, trout and red sea bream has become quite popular but it is known to be difficult to imitate the colors of natural fishes due to differences in living environments and feeds. The reddish tints of the meats and skins of such fishes are closely related with astaxanthin, a pigment which improves the colors of such fishes with a simultaneous improvement of egg quality, hence feeds containing this pigment are being used increasingly.

Since the latter half of the 1970s, researches about astaxanthin-producing *Phaffia rhodozyma* yeast have been made extensively as a source of astaxanthin and its color improving effect has been confirmed. (For example, Ellic A. Johnson et al.: Aquaculture, Vol. 20, p. 123-134 (1980), Anastacia Jentols et al.: The Progressive Fish Culturist, Vol. 53, p. 1-6 (1991), Japanese Laid-open Patent Publication No. 206342/'82, Japanese Laid-open Patent Publication No. 2280064/'92.)

When the *Phaffia rhodozyma* yeast is handled on a commercial scale, problematic is poor stability of astaxanthin to heat and oxygen. Carotenoids including astaxanthin are, regardless of their kind, extremely sensitive to heat and oxygen, which results in poor stability even in an almost pure state. Its instability is sometimes still more marked when it exists in animal or plant cells. Astaxanthin in *Phaffia rhodozyma* yeast, too, is very poor in stability and is easily decomposed. It is, therefore, very difficult to protect it from decomposition during transportation or storage or handling, or during pelletizing at a high temperature.

As a general method of protecting astaxanthin and other carotenoids in a yeast, readily conceivable is a method of coating the yeast with a coating medium or a method of adding an antioxidant within a permissible concentration (for example, Japanese Laid-open Patent Publication No. 206342/'82, Japanese Laid-open Patent Publication No. 48874/'74), but after various studies, the present inventors found it to be extremely difficult to stabilize astaxanthin in *Phaffia rhodozyma* yeast.

When it is handled on a commercial scale, spray-dried cells of *Phaffia rhodozyma* yeast are preferably used but normally obtained dry celles are fine aggregates relatively wide in a range of a particle size of from several micron meters to 100 micron meters, which are poor in fluidity and easy to dust, and thus it can never be said that the powder characteristics are favorable. It is generally conceivable to improve powder characteristics by granulation but granules are sometimes broken during transportation or storage to produce fine powder and thus stronger granules are desired.

Thus, in handling *Phaffia rhodozyma* yeast on a commercial scale, it is an extremely important technical problem to stabilize carotenoids such as astaxanthin in yeast and to obtain strong yeast granules and no preferable methods have been found out yet.

An object of the present invention is to develop simply and cheaply strong *Phaffia rhodozyma* granules through the stabilization of carotenoids such as astaxanthin.

After repeated studies about how to protect decomposition of carotenoids such as astaxanthin in *Phaffia rhodozyma* yeast and how to improve the strength of granules, the present inventors have found out that the intended *Phaffia rhodozyma* yeast is obtainable by drying in the presence of sugar.

### DISCLOSURE OF THE INVENTION

That is, the present invention relates to *Phaffia rhodozyma* yeast stabilized by drying in the presence of sugar and, further, to granules of such *Phaffia rhodozyma* yeast.

### BEST MODES OF PRACTICING THE INVENTION

Hereinafter the present invention will be described in greater detail.

*Phaffia rhodozyma* yeasts usable in the present invention may be of any strain. Yeasts in a log phase, a deceleration phase or a stationary phase are all usable. Also usable are yeasts not subjected yet to any thermal influences, ripened yeasts (yeasts whose metabolic activities were sustained for a while in the absence of or in the presence of an extremely low concentration of a carbon source required for the cultivation), yeasts with their cell walls digested to some extent by self-digestion, yeasts killed by heating, or mixtures of those yeasts. Those yeasts may be either live or dead.

Further, the aforementioned yeasts with their cell walls chemically treated, biologically treated or mechanically treated may be also used. As methods of treating the cell walls of yeast chemically, treating with inorganic acids such as hydrochloric acid, sulfuric acid etc., or treating with hydroxides such as sodium hydroxide, potassium hydroxide etc., may be most general methods used from old. For example, Ellic A. Johnson et al.: Journal of Applied Biochemistry, Vol. 1, p. 273-282 (1979) describes a method of, for example, treating with sulfuric acid. Also known are methods of treating with other chemicals. As biological treating methods, known are methods using such enzymes as lysozyme, *Bacillus circulance* etc., as described in Japanese Patent Publication No. 61907/'88. As a mechanical treating method, a method using a beads mill, a homogenizer or the like has been known from old. Of course, methods combining those treating methods are also usable. It is, of course, possible to use yeasts microcapsulating substances having desired characteristics.

As forms usable, there are included a culture broth or the aforementioned treated solutions as they are, a culture broth with culture medium components eliminated by washing or the aforementioned treated solutions with reagents eliminated by washing, those concentrated or wet yeasts obtained by such general methods as using a centrifugal separator or a filter, or suspensions of dried yeasts.

As sugars usable in the present invention, there are included, glucose, fructose, lactose, maltose, starch sugar, mannitol, sorbitol, galactose, sucrose etc., and it does not matter whether *Phaffia rhodozyma* can assimilate or not. Those are used as they are, aqueous solutions thereof, or as melted from sugar hydrates. Of these, most preferable is glucose as a cheap reducing sugar. A mixture of two or more of the aforementioned sugars is, of course, usable. The proportion of the sugar to a dry yeast is not less than about 1 weight %, preferably about 5-40 weight %, the optimum proportion being thus selected with the cost taken into consideration.

As a method of using sugars, particularly reducing sugars such as glucose etc., as a coating medium, there is known a method of cross-linking a carbonyl group of sugar and a free amino portion of a gelatin molecule to thus form a sugar gelatin matrix and then forming into insoluble beads, as described in Japanese Laid-open Patent Publication No. 258807/'88, but this relates to gelatin granules of calotenoids and is different from the present invention relating to yeasts. As a result, in the present invention it is not absolutely necessary to use both sugar and gelatin.

Sugars may be added to a suspension containing the aforementioned *Phaffia rhodozyma* yeast or to a suspension of a wet yeast or a dry yeast. It is also possible to mix a wet yeast with sugar or a sugar aqueous solution, or a dry yeast with a sugar aqueous solution.

It is also included in the present invention that at a proper time before or after the stoppage of cultivation of the *Phaffia rhodozyma* yeast, sugar usable as a carbon source was allowed to be remainied in the culture broth or sugar added thereto was allowed to be present more than the predetermined level was then dried and/or granulated.

There is no particular limitations with regard to temperature, stirring, mixing conditions etc, but when solids such as powder and granule of sugar are used, heating may be made, or stirring or mixing intensity may be raised for enhancing dissolution. The timing of adding sugar is not particularly limited. Sugar, an aqueous solution of sugar and a melted sugar hydrate may be added intermittently or continuously.

Drying of a mixture of yeast and sugar may be made by a generally known method by the use of a spray drier, a drum drier, freeze drier, a fluidized drier, a spray drier with a built-in fluidized bed, a vacuum drier or the like.

Generally, drying may be conducted under normal pressure or reduced pressure to not more than 15 weight % in a water content, preferably not more than 10 weight % and still more preferably not more than 6 weight %. Drying by means of microwave is also feasible. It is also possible to dry at a temperature of not more than the room temperature but normally the drying temperature is not less than 40°C , and about 100 °C or above in a short period of time is also preferable. It may be also possible to cause a variation of powder strength, water solubility etc., by conducting heat treatment at a relatively high temperature.

The yeast of the present invention can, if necessary, be separated from large or fine particles before, after or during drying. Separation may be carried out by a known method such as passing through a sieve or classification by the use of air current. It is also possible to incorporate a crushing step. Needless to say, it is advantageous to adjust spraying conditions, drying conditions, granulating conditions and crushing conditions so that a preferable range of the particle size is obtainable.

Granulation can be made by a generally known method such as a fluidized bed granulating machine. Any of a method of granulating after drying, drying after granulation and simultaneously conducting drying and glanulation may be adopted. A method of spraying a dry yeast with an aqueous solution of sugar for drying and glanulation may be also adopted. The thus obtained dry yeast is presumable coated partly or wholly with sugar.

In practicing the present invention, it is possible to conjointly use starch, gelatin, gum arabic, dextrin, pectin, pullulan, casein, casein compounds, dry albumin, milk etc.

If necessary, polyvinyl alcohol, polyvinyl pyrrolidone, methyl cellulose, carboxyl methyl cellulose, hydroxyl propyl cellulose, glycerin fatty acid esters, higher alcohols, higher fatty acids, sugar esters of higher fatty acids, hardened oil, shellac, alginates etc., phospholipids such as recithin, emulsifiers such as sorbitan fatty acid esters, polyoxyethylene sorbitan fatty acid esters, polysorbate, alphatocopheryl polyethylene glycol succinate, vitamins such as Vitamin A and Vitamin E, edible oils such as peanut oil and soybean oil, stabilizers such as ethoxyquin, butyl hydroxy anisol and Vitamin C, mold-proof agents such as propionates and acetone, ethanol and methylene chloride as solvents therefor. These may be used singly or in combination of two or more.

As a method of using those substances, there are included a method of further coating and/or granulating a yeast, dried and/or granulated with sugar, with the aforementioned substances, a method of further drying and/or granulating a yeast, coated and/or granulated with the aforementioned substances, with sugar, and a method of drying and/or granulating while adding sugar and the aforementioned substances simultaneously (for example, when sugar and the aforementioned substances are contained in a yeast suspension, when the aforementioned substances are included in an aqueous solution of sugar, or when the individual solutions are added at the same time), any of these methods being usable without any limitation.

Thus, *Phaffia rhodozyma* yeast of the present invention is obtainable very simply and, compared with cases where other coating media or binders are used, is effective to protect astaxanthin from decomposition and is excellent in the strength of granules. The sugar usable in the present invention has no problem about safety as it is used in fields of feeds and foods, and is very easy to handle since it is readily soluble in water and does not require use of any organic solvent dangerous and harmful. Furthermore, it is very cheap and easy to obtain, hence there is no problem about use as a material of the commercial production.

Hereinafter, Reference Examples, Examples, Contrast Examples and Comparative Examples will be given for further description of the present invention in greater detail but, needless to say, the present invention is not limited thereby.

In the following description, "%" means "weight %" unless otherwise noted.

### Reference Example 1: Preparation of dry cell bodies

*Phaffia rhodozyma* yeast was inoculated in 4 large test tubes, each containing 5 ml of a culture medium, and cultured at 20 °C for 48 hours. The contents were then shifted to 4 Sakaguchi flasks, each having a capacity of 500 ml and containing 50 ml of a culture medium, and then cultured at 20°C for 48 hours. The contents were shifted into a mini-jar having a capacity of 5,000 ml containing 2,500 ml of a culture medium and the regular cultivation was carried out at 20 °C. In the regular cultivation, pH was controlled between 4.8 and 5.5 and the dissolved oxygen concentration was maintained between 30% and 80% of saturation. Glucose as a carbon source was added in an amount of 50g at the time of starting and it was fed after consumption thereof. The feeding in an early stage was made slowly and thereafter the feeding rate was made gradually higher not to cause the residue of glucose.

### (Culture medium)

KH₂ PO₄ : 0.7%, (NH₄)₂HPO₄ : 1.3%, MgSO₄ · 7H₂ O: 1530 ppm, ZnSO₄ · 7H₂O: 170 ppm, MnSO₄ · 4H₂O: 17 ppm, NaCl: 170 ppm, CaCl₂ · 2H₂O: 300 ppm, FeSO₄ · 7H₂O: 30 ppm, CuSO₄ · 5H₂O: 1.5 ppm, yeast extract: 0.3%,
Defoaming agent: 100 ppm, pH adjustment : with ammonia water
Addition of glucose was stopped after the cultivation for about 160 hours. 10 hours later, after the adjustment of pH to 7 with an aqueous solution of sodium hydroxide, the yeast was killed at 50°C. Thereafter, the cell bodies were centrifugally separated and collected and then washed with a sufficient amount of water. The cell bodies were added to water and about 10% cell bodies suspension was prepared.

The suspension was spray-dried by the use of a spray drier (inlet temperature 170°C ) and dry cell bodies were obtained. The water content of the dry cell bodies obtained was 6%.

### Reference Example 2: Preparation of suspension of cell bodies

Cultivation was made in the same way as in Reference Example 1 and cell bodies were collected. After acidic hydrolysis at 70°C for 1 hour in a sulfuric acid of 2.5N, the cell bodies were neutralized in an aqueous solution of sodium hydroxide. Thereafter, the cell bodies were centrifugally separated and collected, then washed with a sufficient amount of water. The cell bodies were added to water and about 8% cell bodies suspension was prepared.

### Examples 1-8

40g of dry cell bodies obtained in Reference Example 1 was put into an air current fluidizing and granulating device, and solutions of various coating media were sprayed, coated and granulated. After vacuum drying at room temperature, yeast granules were obtained. The particle sizes were invariably about 50-600 µm. Further, granules of 255-500 µm in particle size were obtained by sifting. The water contents of the granules were 6-8% in all cases.

**Table 1**

| No. | Kind of coating medium | Amount of coating medium (g)/solvent (ml) |
|---|---|---|
| Example 1 | sucrose | 4 g/40 ml (water) |
| Example 2 | glucose | 4 g/40 ml (water) |
| Example 3 | dextrin | 4 g/40 ml (water) |
| Example 4 | gelatin | 4 g/80 ml (water) |
| Example 5 | corn starch | 4 g/40 ml (water) |
| Example 6 | casein sodium | 4 g/40 ml (water) |
| Example 7 | dry albumin | 4 g/40 ml (water) |
| Example 8 | shellac | 8 g/19 ml (ethanol) |

### (Air current fluidizing and granulating device)

Device: Flow Coater "NINI" manufactured by Froint Sangyo Kabushiki Kaisha
Spray dial: ON/OFF = 0.5/0.5
Pulse jet dial: ON/OFF = 0.5/0.5
Air current inlet temperature: 90 °C
Air current outlet temperature: Not definite (about 40-50°C)
Spray pressure: 0.7-1.0 kg/cm²
Air current control dial: 60-80
Spray speed : about 1 ml/min.

The granules obtained under the aforementioned conditions were kept in air at 100°C for 2 hours or at 40 °C for 7 days and stabilities (residual ratios of astaxanthin) were studied. The results are shown in Table 2. As seen from Table 2, the products of the present invention were highly stable.

**Table 2**

| No. | Kind of coating medium | 100°C, 2 hours (%) | 40°C, 7 days (%) |
|---|---|---|---|
| Example 1 | sucrose | 70 | 92 |
| Example 2 | glucose | 78 | 93 |
| Example 3 | dextrin | 68 | 90 |
| Example 4 | gelatin | 61 | 89 |
| Example 5 | corn starch | 65 | 89 |
| Example 6 | casein sodium | 61 | 87 |
| Example 7 | dry albumin | 62 | 87 |
| Example 8 | shellac | 63 | 88 |

### Examples 9-12, Comparative Example 1

Glucose and sucrose in amounts shown in Table 3 were dissolved in about 10 % cell bodies suspensions obtained in the same way as in Reference Example 1. Thereafter the suspensions were spray-dried in the same way as described in Reference Example 1 and dry cell bodies were obtained. The water contents of the dry cell bodies thus obtained were 6-7% in all cases.

**Table 3**

| No. | Kind of sugar | Ratio of sugar to cell bodies(dry basis) (%) |
|---|---|---|
| Example 9 | glucose | 5 |
| Example 10 | glucose | 10 |
| Example 11 | glucose | 20 |
| Example 12 | sucrose | 10 |
| Comp.Example 1 | none | 0 |

The dry cell bodies obtained in the way as described above were stored in air at 100 °C for 2 hours and at 40°C for 7 days and their stabilities (residual ratios of astaxanthin) were studied. The results are shown in Table 4.

As seen from Table 4, the products of the present invention were highly stable.

**Table 4**

| No. | Kind of sugar | Amount added (%) | 10 °C, 2hours (%) | 40°C,7days (%) |
|---|---|---|---|---|
| Example 9 | glucose | 5 | 90 | 92 |
| Example 10 | glucose | 10 | 94 | 92 |
| Example 11 | glucose | 20 | 94 | 93 |
| Example 12 | sucrose | 10 | 93 | 92 |
| Comp.Example 1 | none | 0 | 86 | 90 |

### Example 13, Comparative Example 2

1.3 kg of dry cell bodies obtained in the same way as described in Referemce Example 1 was charged into a spray drier with a built-in fluidized bed. With hot air being supplied, the liquids shown in Table 5 were sprayed at a rate of 5-10 g/min at an intra-bed temperature of 40 °C and granulated and dried. The granules obtained were 200-300 µm and 4-6% in water content in all cases.

**Table 5**

| No. | Spray liquid |
|---|---|
| Example 13 | glucose 60 g/water 600 g solution |
| Comp.Example 2 | water 600 g |

The granules obtained in the way as described above were stored in air at 100°C for 2 hours or at 40 °C for 7 days and their stabilities (residual ratios of astaxanthin) were studied. The results are shown in Table 6. As apparent from the results in Table 6, the product of the present invention has a high stability. When the strength of the granules was evaluated by 3 panelers by finger touch, all panelers judged that the product of the present invention was stronger.

**Table 6**

| No. | 100°C,2 hours (%) | 40 °C,7 days (%) |
|---|---|---|
| Example 13 | 80 | 95 |
| Comp.Example 2 | 73 | 91 |

### Example 14, Comparative Example 3

Glucose in an amount shown in Table 7 was dissolved in the cell bodies suspension obtained in Reference Example 2 and a dry cell bodies were obtained by spray drying as in Reference Example 1. The water contents of the resulting dry cell bodies were 6%.

**Table 7**

| No. | Ratio of glucose to cell bodies(dry base)(%) |
|---|---|
| Example 13 | 20 |
| Comp.Example 3 | 0 |

The aforementioned dry cell bodies were stored in air at 100 °C for 2 hours or at 40 °C for 7 days and their stabilities (residual ratios of astaxanthin) were studied. The results are shown in Table 8. As seen from Table 8, the product of the present invention was quite high in stability.

**Table 8**

| No. | 100°C, 2 hours (%) | 40°C, 7 days (%) |
|---|---|---|
| Example 13 | 83 | 91 |
| Comp.Example 3 | 65 | 86 |

The aforementioned dry cell bodies were put into a aluminium laminated bag and heat-sealed in air and another laminated bag containing the same was heat-sealed after perfect substitution with nitrogen. These were stored at 25 °C for 1 month and their stabilities (residual ratios of astaxanthin) were studied. The results are shown in Table 9. As is apparent from the results in Table 9, the product of the present invention was quite high in stability.

**Table 9**

| No. | In air (%) | In nitrogen substituted (%) |
|---|---|---|
| Example 13 | 92 | 94 |
| Comp.Example 3 | 86 | 92 |

### Examples 14-16, Comparative Example 4

20 g of dry cell bodies obtained in the same way as described in Reference Example 1 was suspended in 100 ml of water. Glucose in amounts given in Table 10 was dissolved in the suspensions. The suspensions were then vacuum-dried for 3 days. After crushing, those were sifted and powders of 355-600 µm in particle size were obtained. Those were further vacuum-dried for 1 day at a temperature in a range of 20-40 °C. The water content was 7-13% in every case.

**Table 10**

| No. | Ratio of glucose to cell bodies(dry basis)(%) |
|---|---|
| Example 14 | 6 |
| Example 15 | 15 |
| Example 16 | 40 |
| Comp.Example 4 | 0 |

The granules obtained in the way described above were stored in air at 100 °C for 2 hours or at 40 °C for 7 days and their stabilities (residual ratios of astaxanthin) were studied. The results are shown in Table 11. As is apparent from the results in Table 11, the products of the present invention were quite high in stability.

**Table 11**

| No. | Amount of glucose (%) | 100°C, 2hours (%) | 40°C, 7 days (%) |
|---|---|---|---|
| Example 14 | 6 | 79 | 91 |
| Example 15 | 15 | 90 | 94 |
| Example 16 | 40 | 95 | 97 |
| Comp.Example 4 | 0 | 55 | 90 |

### Examples 17-20, Comparative Example 5

10 g of dry cell bodies obtained in Example 1 was charged into a container having a capacity of water 100 ml. With this container being shaked violently, the liquids shown in Table 12 were dropped thereinto. The granules were sifted to have their particle sizes adjusted to 250-600 µm. Those were then vacuum-dried at room temperature over about 20 hours. The water content of the resulting granules was 5-7% in every case.

**Table 12**

| No. | Liquid dropped |
|---|---|
| Example 17 | glucose 0.1 g/water 3 ml solution |
| Example 18 | sucrose 0.1 g/water 3 ml solution |
| Example 19 | methyl cellulose 0.1 g/water 6 ml solution |
| Example 20 | dextrin 0.1 g/water 3 ml solution |
| Comp.Example 5 | water 3 ml |

The strength of the granules obtained in the aforementioned Examples 17-20 and Comparative Example 5 was evaluated by 4 panelers (A, B, C, D) by finger touch method.

The results are as shown in Table 13. From the results in Table 13, it is understood that the products of Examples 17-20, especially Examples 17 and 18, were quite strong.

**Table 13**

| No. | A | B | C | D |
|---|---|---|---|---|
| Example 17 | Difficult to break | Difficult to break | Difficult to break | Difficult to break |
| Example 18 | Difficult to break | Intermediate | Difficult to break | Difficult to break |
| Example 19 | Intermediate | Impossible to evaluate | Intermediate | Intermediate |
| Example 20 | Intermediate | Intermediate | Intermediate | Intermediate |
| Comp. Example 5 | Easy to break | Easy to break | Easy to break | Easy to break |

In the avove Table 13, "Intermediate" means an intermediate level between "Difficult to break" and "Easy to break".

### Examples 21-22, Contrast Example 1, Comparative Example 6

Granules of the compositions shown in Table 14 were obtained in the same way as in Example 1.

**Table 14**

| No. | Amount of glucose(%) (to dry cell bodies) | Amount of antioxidant(%) (to dry cell bodies) |
|---|---|---|
| Example 21 | 20 | Vitamin C sodium 1 |
| Example 22 | 20 | ethoxyquin 0.1 |
| Cont.Example 1 | 20 | 0 |
| Comp.Example 6 | 0 | 0 |

The aforementioned granules were stored in air at 100°C for 2 hours or at 40 °C for 7 days and their stabilities (residual ratios of astaxanthin) were studied. The results are shown in Table 15. As seen from the results in Table 15, the products of Examples 21, 22 and Contrast Example 1, Examples 21 and 22 in particular, were quite high in stability.

**Table 15**

| No. | 100 °C, 2 hours(%) | 40 °C, 7 days (%) |
|---|---|---|
| Example 21 | 86 | 96 |
| Example 22 | 85 | 95 |
| Cont.Example 1 | 81 | 94 |
| Comp.Example 6 | 64 | 90 |

### Examples 23-27

Granules of the compositions shown in Table 16 were obtained in the same way as in Example 1.

**Table 16**

| No. | Amount of glucose(%) | Amount of other additives(%) |
|---|---|---|
| Example 23 | 10 | dextrin 5 |
| Example 24 | 10 | pullulan 3 |
| Example 25 | 20 | corn starch 5 |
| Example 26 | 20 | lecithin 5 |
| Example 27 | 20 | casein sodium 3 |

### POSSIBILITY OF THE INDUSTRIAL UTILIZATION

According to the present invention, granules which are highly effective for protection from decomposition of astaxanthin and high in granulation strength can be obtained easily and cheaply.

## Claims

1. *Phaffia rhodozyma* yeast with the contained astaxanthin stabilized, which is dried in the presence of sugar.

2. *Phaffia rhodozyma* yeast according to Claim 1, wherein the sugar comprises at least one kind selected from the group consisting of sucrose and glucose.

3. *Phaffia rhodozyma* yeast according to Claim 1 or 2, wherein not less than 1 weight % of sugar is present based on the yeast (dry basis) contained.

4. *Phaffia rhodozyma* yeast according to Claim 3, wherein not less than 5 weight % of sugar is present based on the yeast (dry basis) contained.

5. Granules of *Phaffia rhodozyma* yeast according to claims 1-4.
